(19) Europäisches Patentamt
European Patent Office
Office européen des brevets

(11) Publication number : **0 386 657 B1**

# (12) EUROPEAN PATENT SPECIFICATION

(45) Date of publication of patent specification : **13.01.93 Bulletin 93/02**

(51) Int. Cl.$^5$ : **A61K 31/70**

(21) Application number : **90104128.5**

(22) Date of filing : **02.03.90**

## (54) Preparation for treating systemic lupus erythematosus.

(30) Priority : **08.03.89 JP 55562/89**

(43) Date of publication of application : **12.09.90 Bulletin 90/37**

(45) Publication of the grant of the patent : **13.01.93 Bulletin 93/02**

(84) Designated Contracting States : **BE CH DE DK FR GB IT LI LU NL SE**

(56) References cited :
**EP-A- 0 296 620**
**TOHOKU J. EXP. MED., vol. 114, no 4, 1974, pages 339-348; T. KUROYANAGI et al.: "Relationship between contents of neuramini-cacid and ATP and adhesiveness of the platelet"**
**J. UOEH, vol. 7, no. 4, 1985, pages 401-407; T. OTA et al.: "Clinical significance of serum sialic acid in rheumatoidarthritis and systemic lupus erythematosus"**
**RHEUMATOL INT., vol. 2, no. 4, 1982, pages 145-149; C.P.J. MAURY et al.: "Serum beta2-microglobulin, sialic acid, andC-reactive protein in systemic lupus erythematosus"**

(73) Proprietor : **MECT CORPORATION**
**2-1-1, Nishi-Shinjyuku Shinjyuku-ku**
**Tokyo (JP)**

(72) Inventor : **Miyata, Takeshi**
**2178-64 Nagaminemachi Kumamoto-shi**
**Kumamoto (JP)**
Inventor : **Takahama, Kazuo**
**19-3 Magano Matsubase-machi**
**Shimomachiki-gun**
**Kumamoto (JP)**
Inventor : **Kai, Hirofumi**
**Hakuzan-Shukusya 3-7 6-54 Hakuzan 1-chome**
**Kumamoto-shi Kumamoto (JP)**
Inventor : **Ishii, Takayuki**
**2-19-4 Hikawadai Nerima-ku**
**Tokyo (JP)**
Inventor : **Komatsu, Kenji**
**c/o MECT Corporation 1-1 Nishi-Shinjuku**
**2-chome**
**Shinjuku-ku Tokyo (JP)**
Inventor : **Ogasawara, Sadanori**
**900-14 Arahata Tokorozawa-shi**
**Saitama (JP)**

(74) Representative : **Berendt, Thomas, Dr.rer.nat. Dipl.-Chem. et al**
**Patentanwälte Dr.rer.nat. Dipl.-Chem. Th. Berendt Dr.Ing. Hans Leyh Dipl.-Ing. Hartmut Hering Innere Wiener Strasse 20**
**W-8000 München 80 (DE)**

**EP 0 386 657 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a preparation for treating systemic lupus erythematosus, more particularly to a preparation containing N-acetylneuraminic acid as an effective substance.

In vivo, a sialic acid combines with heteropolysaccharides in various ways and carries biological functions as an important constituent for the heteropolysaccharides.

Regarding biological functions of the sialic acid in an immune system, there have heretofore been carried out many researches thereon, and have been reported that it shows specific antigenic property, is essential component for receptor molecule, or shows anti-recognition effect which masks immune recognition site.

In recent years, there has been reported that N-acetylneuraminic acid which is one of the most representative sialic acids shows antiviral effect or anti-inflammatory effect [(1) Robert L.Hirsch, et al., The Journal of Immunology, vol. 127, No. 5 (1981), pp. 1740-1743;(2) P. Gorog, et al., Agents and Actions, vol. 8, No. 5 (1978), pp. 543-545; (3) Hiromi Itoh, et al., Pharmacology and Therapy, vol. 13, No. 7 (1985), pp. 479-494, et al].

## SUMMARY OF THE INVENTION

The present inventors have investigated concerning the pharmacological effect of sialic acid for a long time, and have found that N-acetylneuraminic acid which is a representative compound thereof has inhibiting effect to Arthus reaction.

An object of the present invention is to provide a preparation for treating immune systemic lupus erythematosus containing N-acetylneuraminic acid or salt thereof represented by the following formula:

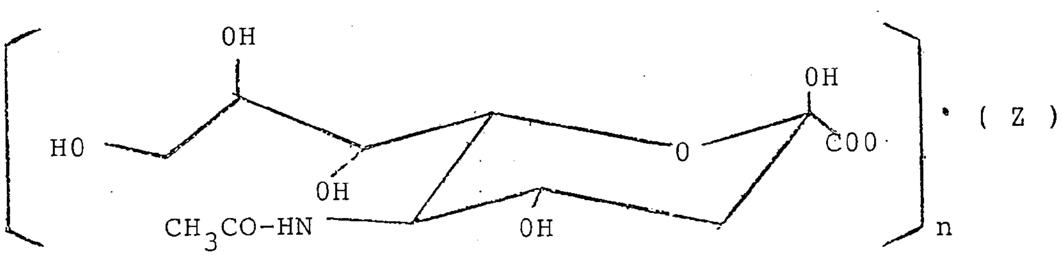

wherein, when n is 1, Z represents a hydrogen, lithium, potassium, sodium, ammonium or organic ammonium, and when n is 2, Z represents calcium, barium or magnesium,
as an active ingredient.

The Arthus reaction is an inflammation reaction which can be observed particularly at skin as edema and bleeding, and its representative disease is systemic lupus erythematosus (SLE). Other diseases of the Arthus reaction are serum disease, complex glomerular nephritis, etc.

Main reaction procedures of the Arthus reaction are (1) sedimentation of immune complex at vessel wall and surrounding tissues, (2) activation of complement system through classical pathway (partially, through the alternative pathway), (3) aggregation of polymorphonuclear leukocytes to a reaction site, (4) immune adhesion of polymorphonuclear leukocytes to materials and cells in which C3b is coated thereon, (5) reinforcement of phagocytosis of polymorphocuclear leukocytes, (6) release of lysosomal enzyme from plymorphonuclear leukocytes, and (7) disorder of cells (erythrocytes, etc.) and tissues.

N-acetylnueraminic acid and its salts, in vivo, show inhibitory effect to bleeding due to above Arthus reaction depending on amount to be used.

N-acetylneuraminate can be obtained, for example, by neutralizing N-acetylneuraminic acid with hydroxide or carbonate of alkali metal or alkaline earth metal, and separating salt from reactant (Japanese Patent Application No. 61-51898).

## BRIEF DESCRIPTION OF THE DRAWINGS

Fig. 1 is a graphical representation of effects of N-acetylneuraminate against passive Arthus reaction, showing diameters of bleedings measured after 2 hours of antigen challenge, and

Fig. 2 is a graphical representation of effects of N-acetylneuraminate agaist passive Arthus reaction, showing diameters of bleedings measured after 24 hours of antigen challenge.

## DETAILED DESCRIPTION OF THE PREFERRED EMBODIMENTS

Example 1

Effects on N-acetylneuraminate against passive Arthus reaction

(1) Animal for experiment

Male Hartley guinea pig weighing 300 to 400 g was used.

(2) Preparation of antiserum (rabbit heterocellular affinity antiserum)

According to the conventional method, Anti-OA (rabbit) was prepared. That is, by using New Zealand strain rabbits having a weight of 2.0 to 2.2 kg, 1 ml of an emulsion comprising 20 mg of OA and Freund complete adjuvant (available from DIFCO LABORATORIES) was subcutaneously injected to back side portion and active sensitization was effected for total 4 times for every two weeks.

After two weeks from the final sensitizing date, blood was gathered from common carotid artery and after separation of antiserum, it was stored under freezing at -20°C by the usage for experiment.

(3) Experimental method

The passive Arthus reaction was effected according to the method of Katayama et al. [S. Katayama, et al., Arzneim.-Forsh., vol. 31, p. 1196 (1981)]. However, in order to cause sufficient reaction, change were added to a concentration of OA solution. That is, sensitization was effected by intravenously injecting 1 ml of antiserum stock solution and 30 minutes after, the reaction was caused by intradermally injecting respective 0.05 ml of OA solutions, each containing 0.025 mg and 0.05 mg of OA respectively, to each one portion of an abdominal part and total two portions of guinea pig at which hair was cut at the day before. At this time, a physiological saline solution was intradermal injected simultaneously in the same manner as a blank. After 2 hours and 24 hours of challenge of antigen, a diameter of bleeding at each stimulated portion was measured and a square of the diameter was made as an index of a degree of the reaction. And, the value was calibrated by the blank to be made as the experimental results.

1, 5 or 20 mg/kg of sodium N-acetylneuraminate, or physiological saline solution (1 mg/kg) as a control was intravenously injected before 30 minutes of antigen-challenge or immediately after sensitization.

(4) Statistical treatment

Experiments more than 6 were carried out and the resulting experimental results were shown as an average ± standard deviation. Also, Student's t-test was employed for significant test and $p < 0.05$ was made as statistically significant.

Experimental results are shown in Fig. 1 and Fig. 2.

(5) Results

As clearly seen from Fig. 1 and Fig. 2, by administering sodium N-acetylneuraminate, bleeding of skin can be depressed depending on an amount to be used, and it is remarkable at 20 mg/kg. Also, this inhibiting effect is continued even after 24 hours from antigen challenge.

Example 2

Acute toxicity test

Acute toxicity tests of sodium N-acetylneuraminate against mouse, rat and guinea pig due to oral, subcutaneous injection, intraperitoneal injection, intravenous injection and inhalation were effected as shown below.

(1) Animals to be tested

| | |
|---|---|
| ICR mouse | 6 weeks old |
| SD rat | 6 weeks old |
| Hartley guinea pig | 6 weeks old |

(2) Chemicals concentration

20 % (w/v) dissolved in distilled water

(3) Animal number per one level
Ten

(4) Terms observed
14 days

(5) Calculation method of $LD_{50}$
Probit method

The results are shown in Table 1.

Table 1

Acute toxicity test of sodium N-acetylneuraminate

| Kinds of animal | sex | $LD_{50}$ (mg/kg) Administration route | | | | |
|---|---|---|---|---|---|---|
| | | p.o. | s.c. | i.p. | i.v. | inhalation |
| Mouse | male | >5,000 | >5,000 | >5,000 | >5,000 | - |
| | female | >5,000 | >5,000 | >5,000 | >5,000 | - |
| Rat | male | >5,000 | >5,000 | >5,000 | >5,000 | >4,000 mg/m$^3$ |
| | female | >5,000 | >5,000 | >5,000 | >5,000 | >4,000 mg/m$^3$ |
| Guinea pig | | - | - | - | >5,000 | - |

p.o.:per os, s.c.:subcutaneously
i.p.:intraperitoneally, i.v.:intravenously

Inhalation is effected by exposing powder to be tested for one hour.

Example 3

Simple acute toxicity test

Simple acute toxicity test of the agent to be tested due to intravenous injection to mouse was effected as

shown below.

1. Experimental material and method

(1) Agents to be tested

Lithium salt, potassium salt, barium salt and magnesium salt of N-acetylneuraminic acid (all available from MECT Corporation) were used.

(2) Animal for experiment

ddy male mouse

Weight at initial of the test : 17.7 to 21.1 g

Animal number per one level : 3

(3) Room temperature : 23 ± 1 °C, Humidity : 55 ± 7 %

(4) Administration route : intravenous

(5) Administration method and amount thereof

The above agent to be tested was dissolved with a physiological saline solution, and that which is prepared so as to become the administration amount of 0.2 ml per 20 g of mouse weight was injected into a tail vein. An administered amount was made to three levels of 500, 1000 and 2000 mg/kg.

(6) Observation of general condition and death condition

Observations of the general condition and presence or absence of death were conducted from immediately after administration to after 7 days.

2. Results

(1) Death rate

Death rate was shown in Table 2.

At 1000 and 2000 mg/kg, 3 per 3 samples were died in magnesium salt, barium salt and potassium salt of N-acetylneuraminic acid. At 500 mg/kg, 3 per 3 samples were died in N-acetylneuraminic acid barium salt. Other than the above, no death was observed.

(2) General condition

Died samples were accompanied by clonic convulsion and incontinence of urine and almost all the samples died immediately after administration or within one minute. In the viral samples, inhibition in ultromotivity can be observed in a little sample but recovered within one hour.

Table 2

Died number with a lapse of time

| Agent | Administerd amount mg/kg | 1 | 3 | 6 | 24 hrs | 2 | 3 | 4 | 5 | 6 | 7 days | Final death rate |
|---|---|---|---|---|---|---|---|---|---|---|---|---|
| Lithium N-acetylneuraminate | 500 | 0 | 0 | 0 | 0 | 0 | 0 | 0 | 0 | 0 | 0 | 0/3 |
| | 1000 | 0 | 0 | 0 | 0 | 0 | 0 | 0 | 0 | 0 | 0 | 0/3 |
| | 2000 | 0 | 0 | 0 | 0 | 0 | 0 | 0 | 0 | 0 | 0 | 0/3 |
| Potassium N-acetylneuraminate | 500 | 0 | 0 | 0 | 0 | 0 | 0 | 0 | 0 | 0 | 0 | 0/3 |
| | 1000 | 3 | 0 | 0 | 0 | 0 | 0 | 0 | 0 | 0 | 0 | 3/3 |
| | 2000 | 3 | 0 | 0 | 0 | 0 | 0 | 0 | 0 | 0 | 0 | 3/3 |
| Barium N-acetylneuraminate | 500 | 3 | 0 | 0 | 0 | 0 | 0 | 0 | 0 | 0 | 0 | 3/3 |
| | 1000 | 3 | 0 | 0 | 0 | 0 | 0 | 0 | 0 | 0 | 0 | 3/3 |
| | 2000 | 3 | 0 | 0 | 0 | 0 | 0 | 0 | 0 | 0 | 0 | 3/3 |
| Magnesium N-acetylneuraminate | 500 | 0 | 0 | 0 | 0 | 0 | 0 | 0 | 0 | 0 | 0 | 0/3 |
| | 1000 | 3 | 0 | 0 | 0 | 0 | 0 | 0 | 0 | 0 | 0 | 3/3 |
| | 2000 | 3 | 0 | 0 | 0 | 0 | 0 | 0 | 0 | 0 | 0 | 3/3 |

## Claims

1. Use of a preparation containing an effective amount of a compound represented by the formula:

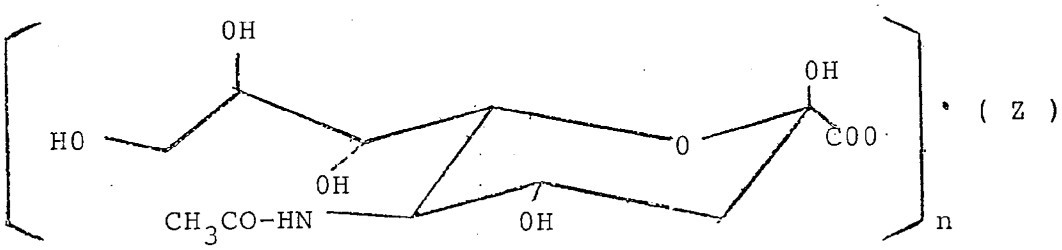

wherein, when n is 1, Z represents a hydrogen, lithium, potassium, sodium, ammonium or an organic ammonium; and when n is 2, Z represents calcium, barium or magnesium as an active ingredient and a pharmacologically permittable carrier, for the manufacture of a medicament for the treatment of systemic lupus erythematosus.

2. Use of the preparation according to claim 1, wherein the preparation is a powder preparation and dissolved when using.

3. Use of the preparation according to claim 1, wherein the preparation is a lyophilized preparation.

4. Use of the preparation according to claim 1, wherein the preparation is a water soluble preparation.

5. Use of the preparation according to claim 1, wherein the preparation contains at least one of agents selected from stabilizers, buffers, isotonic agents, excipients, pH adjusters and preservatives as a carrier.

## Patentansprüche

1. Verwendung einer Präparation, die eine wirksame Menge einer Verbindung enthält, welche durch die Formel gegeben ist:

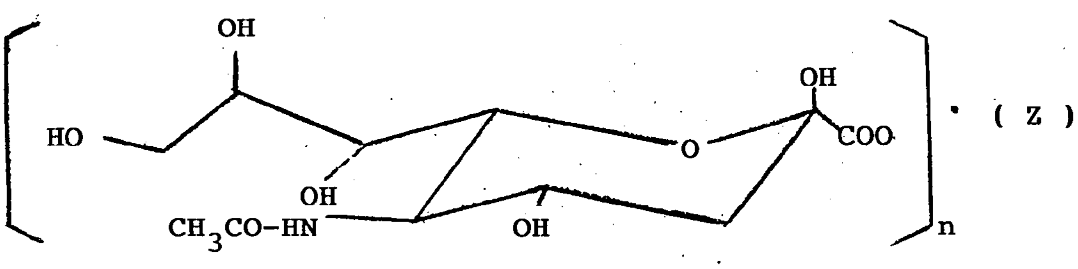

in der, wenn n = 1 ist, Z Wasserstoff, Lithium, Kalium, Natrium, Ammonium oder ein organisches Ammonium darstellt; und wenn n = 2 ist, Z Kalzium, Barium oder Magnesium als aktiven Bestandteil und pharmakologisch unbedenklichen Träger darstellt, für die Herstellung eines Medikamentes für die Behandlung von Schmetterlingsflechte (Lupus Erythematosus).

2. Verwendung der Präparation nach Anspruch 1, **dadurch gekennzeichnet**, daß die Präparation eine pulverförmige Präparation ist, die im Gebrauch aufgelöst ist.

3. Verwendung der Präparation nach Anspruch 1, **dadurch gekennzeichnet**, daß die Präparation eine gefriergetrocknete Präparation ist.

4. Verwendung der Präparation nach Anspruch 1, **dadurch gekennzeichnet**, daß die Präparation eine wasserlösliche Präparation ist.

5. Verwendung der Präparation nach Anspruch 1, **dadurch gekennzeichnet**, daß die Präparation wenigstens ein Reagens enthält,ausgewählt aus Stabilisatoren, Puffern, isotonischen Mitteln, Excipienten, pH-Regulatoren und Konservierungsmitteln als Träger.

## Revendications

1. Utilisation d'une préparation contenant une quantité efficace d'un composé représenté par la formule :

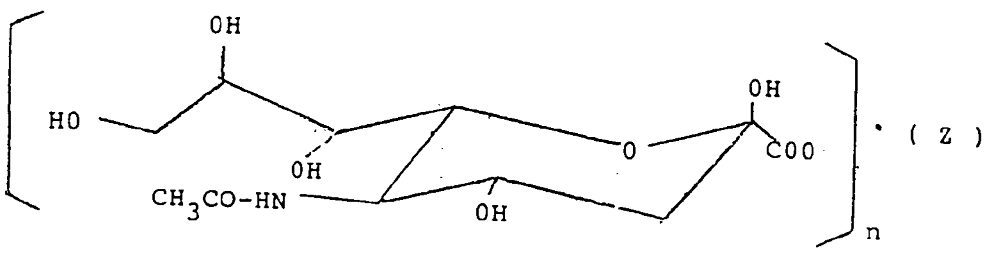

dans laquelle, lorsque n est égal à 1, Z représente un hydrogène, un lithium, un potassium, un sodium, un ammonium ou un ammonium organique, et, quand n est égal à 2, Z représente un calcium, un baryum ou un magnésium en tant qu'ingrédient actif, et un véhicule pharmacologiquement autorisé, pour la préparation d'un médicament pour le traitement du lupus érythémateux systémique.

2. Utilisation de la préparation selon la revendication 1, dans laquelle la préparation est une poudre et est dissoute lorsqu'on l'utilise.

3. Utilisation de la préparation selon la revendication 1, dans laquelle la préparation-est une préparation lyophilisée.

4. Utilisation de la préparation selon la revendication 1, dans laquelle la préparation est une préparation soluble dans l'eau.

5. Utilisation de la préparation selon la revendication 1, dans laquelle la préparation contient comme véhicule, au moins un des agents sélectionnés parmi des agents de stabilisation, des tampons, des agents isotoniques, des excipients, des agents d'ajustement de pH et des conservateurs.

FIG. 1

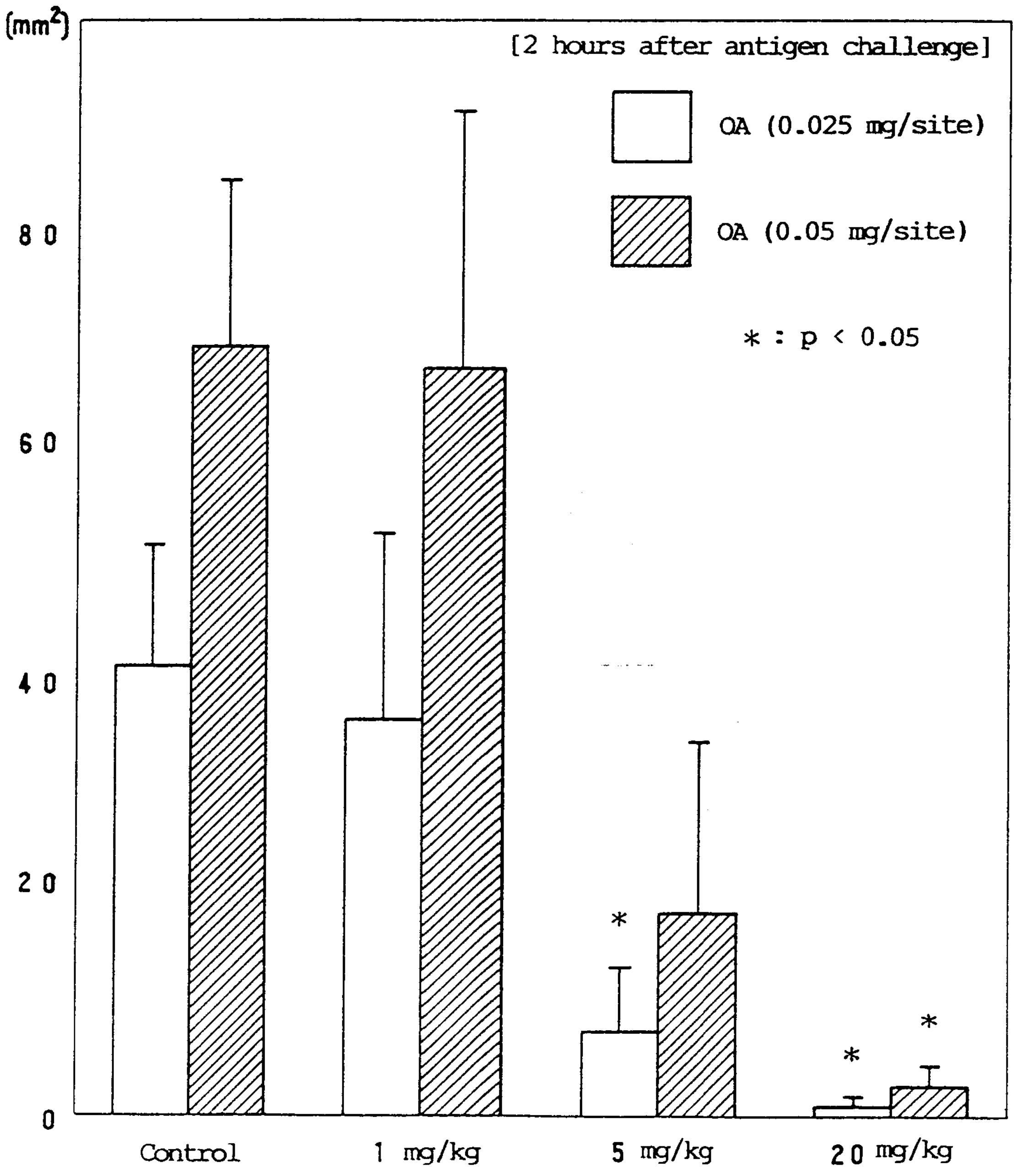
(mm²)
[2 hours after antigen challenge]
OA (0.025 mg/site)
OA (0.05 mg/site)
* : p < 0.05
80
60
40
20
0
*
*
*
Control
1 mg/kg
5 mg/kg
20 mg/kg

FIG. 2

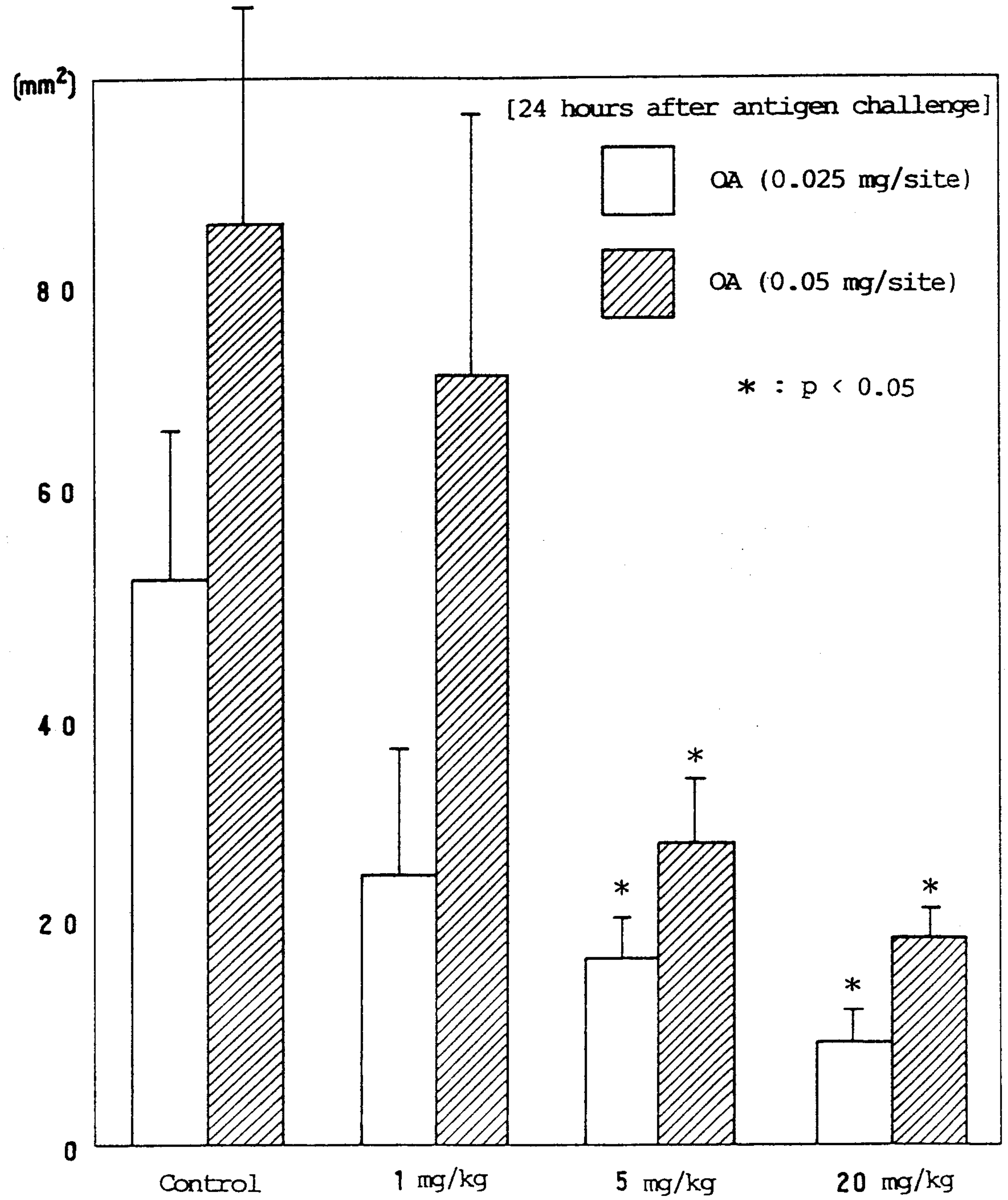
(mm²)
[24 hours after antigen challenge]
OA (0.025 mg/site)
OA (0.05 mg/site)
* : p < 0.05
80
60
40
20
0
*
Control
1 mg/kg
5 mg/kg
20 mg/kg